# FASCICULE DE BREVET EUROPEEN

(11) **EP 1 242 355 B1**
(45) Date de publication et mention de la délivrance du brevet: **25.02.2004**
(21) Numéro de dépôt: 00993709.5
(22) Date de dépôt: 27.12.2000
(51) Int. Cl.: C07C 51/41, C07C 51/43, C07C 55/14, C07C 55/02

(54) **PROCEDE DE FABRICATION DE SEL DE DIAMINE ET DIACIDE CARBOXYLIQUE**
VERFAHREN ZUR HERSTELLUNG EINES DIAMINSALZES VON DICARBOXYLSÄUREN
METHOD FOR PRODUCING DIAMINE AND CARBOXYLIC DIACID SALT

(30) Priorité: 30.12.1999 FR 9916712
(43) Date de publication de la demande: 25.09.2002
(73) Titulaire: Rhodia Polyamide Intermediates, 69192 Saint-Fons (FR)
(72) Inventeur: BISCANS, Béatrice, F-31750 Escalquens (FR); CARVIN, Philippe, F-69005 Lyon (FR); DELMAS, Henri, F-31000 Toulouse (FR); RATSIMBA, Berthe, F-31100 Toulouse (FR); ROQUES, Yves, F-69100 Villeurbanne (FR)
(74) Mandataire: Esson, Jean-Pierre
(86) Numéro de dépôt international: PCT/FR2000/003693
(87) Numéro de publication internationale: WO 2001/049646

(56) Documents cités:
- FR-A- 2 215 411
- US-A- 5 471 001
- US-A- 5 658 534

## Description

La présente invention concerne un procédé de fabrication de sel de diamine et de diacide carboxylique. Plus particulièrement elle concerne la fabrication d'un tel sel à l'état non dissous, et encore plus précisément la fabrication du sel solide d'adipate d'hexaméthylène diamine.

Les procédés de fabrication des polyamides à partir de monomères diacides et diamines utilisent généralement comme matières premières le sel acide de diamine permettant un meilleur contrôle du rapport stoechiométrique entre le diacide et la diamine.

Ainsi, la fabrication du polyamide 6,6 est réalisée à partir de l'adipate d'hexaméthylène diamine également appelé sel Nylon ou sel N.

Ce sel est obtenu généralement par addition d'acide adipique dans une solution aqueuse d'hexaméthylène diamine.

Ce procédé permet d'obtenir une solution aqueuse de sel Nylon de concentration faiblement supérieure à 50 %.

Une telle solution est utilisée dans le cas de production intégrée de polyamide. Elle peut être également transportée sous certaines conditions d'atmosphère neutre et à une température évitant la cristallisation du sel.

Toutefois, quand il est nécessaire de transporter le sel Nylon, il est très avantageux de l'obtenir à l'état solide pour éviter le transport d'eau et les conditions particulières de transport. Plusieurs procédés de fabrication de sel Nylon à l'état solide ont été proposés. Parmi ceux-ci, deux procédés principaux ressortent.

Un premier procédé consiste à dissoudre l'acide adipique dans un alcool tel que le méthanol et à faire de même avec l'hexaméthylène diamine (HMD). Ces deux solutions sont mélangées sous agitation. Comme le sel Nylon est insoluble dans l'alcool, il précipite. Après récupération par exemple par filtration, le sel Nylon recueilli est lavé par l'alcool et séché.

Un tel procédé est décrit notamment dans les brevets FR 1 438 710 ou US 2 130 947.

Le second procédé consiste à réaliser une solution aqueuse de sel Nylon puis à la concentrer à chaud et à la refroidir. Le sel Nylon cristallise. Les cristaux récupérés sont ensuite séchés. La concentration de la solution peut être obtenue par évaporation de l'eau ou selon un autre procédé par addition d'acide adipique dans la solution de sel Nylon puis addition de HMD pour faire précipiter le sel Nylon. Ce procédé est décrit par exemple dans le brevet FR 2 215 411.

La présente invention s'applique plus particulièrement au second procédé de précipitation dans l'eau du sel de diamine après concentration de la solution par divers moyens.

Le sel solide ainsi produit est stable et peut être transporté sans précaution particulière, notamment son transport ne nécessite pas l'emploi d'une atmosphère non oxydante telle que l'azote.

Toutefois, le sel de diamine solide doit être très sec pour limiter les phénomènes de mottage ou agglomération au cours de son stockage ou transport. Ces phénomènes sont très importants et pénalisants, limitant actuellement l'utilisation de sel de diamine solide.

En outre, pour obtenir un polymère de très bonnes caractéristiques physiques, physico-chimiques, il est nécessaire d'utiliser un sel de diamine présentant des critères très élevés de pureté. En conséquence, la précipitation du sel doit être réalisée avec beaucoup de précaution pour éviter notamment l'inclusion de solution mère dans les cristaux. Ce phénomène est actuellement très difficile à éviter.

Un des buts de la présente invention est de proposer un procédé de fabrication de sel de diacide et diamine tels que le sel Nylon à l'état solide présentant des cristaux ou particules de grande pureté et aptes à être stockés et transportés sans mottage.

A cet effet, l'invention propose un procédé de cristallisation de sel de diamine et de diacide carboxylique consistant à soumettre une solution aqueuse dudit sel à une agitation ultrasonique simultanément à une saturation de ladite solution aqueuse en sel.

Par le terme saturation, il faut comprendre tout procédé qui permet de modifier la concentration du sel dans la solution à une valeur compatible avec une cristallisation de celui-ci. Généralement cette concentration est au moins égale et plus préférentiellement supérieure à la concentration de saturation du sel à la température considérée. Plus précisément, cette concentration correspond à une sursaturation de la solution de sel.

La solution aqueuse peut, bien entendu, être avantageusement soumise à une agitation mécanique, telle que celle utilisée dans les installations usuelles de cristallisation.

Le procédé de l'invention permet d'obtenir des cristaux denses et compacts de sel de diamine et diacide carboxylique, c'est-à-dire ne contenant pas ou sensiblement pas d'inclusion de solution mère aqueuse. Ainsi, le sel obtenu a un degré de pureté plus élevé et surtout contient une très faible quantité d'eau diminuant les risques de mottage des cristaux pendant leur stockage ou transport.

Selon une autre caractéristique de l'invention, la saturation de la solution aqueuse en sel peut être obtenue par utilisation de plusieurs procédés mis en oeuvre indépendamment l'un de l'autre ou en combinaison.

Ainsi, le premier procédé, le plus usuel, consiste à dissoudre une quantité de sel N dans de l'eau à une température supérieure à 50°C et voisine de 100°C, puis à refroidir cette solution pour provoquer sa saturation et donc la cristallisation du sel.

Un autre procédé consiste à évaporer l'eau de la solution aqueuse par exemple par mise sous pression réduite de ladite solution, pour saturer la solution et provoquer la cristallisation du sel.

Enfin, dans un troisième mode de réalisation, la saturation de la solution est obtenue par addition successive ou simultanée d'un flux de diacide et d'un flux de diamine dans une solution de sel.

Ces procédés pour réaliser la saturation de la solution de sel ne sont donnés qu'à titre indicatif. Ainsi, le résultat de l'invention sera obtenu quel que soit le procédé de réalisation de la saturation de la solution mise en oeuvre.

De même, l'invention s'applique à la cristallisation de tous sels de diamines et plus particulièrement pour les sels obtenus à partir d'hexaméthylène diamine, et d'acides dicarboxyliques comme l'acide adipique, l'acide décadioïque. De manière préférée, l'invention s'applique à la cristallisation de l'adipate d'hexaméthylène diamine également appelé sel Nylon ou sel N.

Selon une autre caractéristique de l'invention, la solution aqueuse de sel soumise à l'agitation ultrasonique contient avantageusement des cristaux naissants de sel pour ainsi initier la cristallisation de cuilui-ci et limiter les phénomènes de sursaturation.

Ces cristaux naissants peuvent être ajoutés dans la solution ou générés par tous procédés connus.

Selon l'invention, la solution aqueuse, est soumise à une agitation ultrasonique produite par un générateur d'ultrasons dont la ou les sondes peuvent être immergées dans la solution ou disposées sur les parois du cristallisoir contenant la solution, par exemple en formant une de ses parois.

Selon un autre mode de réalisation du procédé de l'invention, la solution de sel est mise en circulation dans un conduit en boucle fermée externe à l'enceinte de cristallisation, l'agitation ultrasonique étant appliquée sur la solution circulant dans le conduit.

Des exemples de procédés et dispositifs d'agitation ultrasonique d'une solution sont décrits dans les brevets US 5,471,001, US 5,395,593 et US 5,658,534. Ces exemples sont donnés uniquement à titre indicatif.

Selon un mode de réalisation préféré de l'invention, l'agitation ultrasonique est obtenue par des ultrasons émis à une fréquence comprise entre 18 KHz et 200 KHz, de préférence entre 20 et 40 KHz.

Cette agitation ultrasonique peut être appliquée de manière continue ou en mode pulsé.

Le générateur d'ultrasons comprend une ou plusieurs sondes plongeantes ou une ou plusieurs sondes comprenant une surface pouvant former la paroi d'un cristallisoir. Les sondes peuvent également être disposées autour ou sur une paroi d'un récipient ou d'un conduit. A titre d'exemple de générateur d'ultrasons, on peut citer les dispositifs commercialisés sous les dénominations commerciales VIBRACELL, SINAPTEC.

D'autres avantages, détails de l'invention apparaîtront plus clairement au vu des exemples donnés ci-dessous uniquement à titre indicatif et des figures annexées dans lesquelles :
- La figure 1 représente une photographie au microscope à balayage électronique des cristaux de sel Nylon obtenus par cristallisation sans agitation ultrasonique,
- La figure 2 représente une photographie au microscope à balayage électronique des cristaux de sel Nylon obtenus par cristallisation avec une agitation ultrasonique de puissance 100 W.

Une solution de sel NYLON est obtenue par solubilisation d'un sel NYLON obtenu par précipitation en milieu aqueux avec évaporation de l'eau, séparation par essorage et séchage.

Les essais de cristallisation sous ultrasons sont réalisés en mode discontinu dans un réacteur du type "Cup-horn", c'est à dire un réacteur dont le fond est formé par la sonde ou sonotrode du générateur d'ultrasons. La fréquence d'émission des ultrasons est de 20 KHz.

Le réacteur ou cristallisoir est équipé avec un agitateur en forme d'hélice marine à 4 pales avec trois contre-pales pour limiter les effets de Vortex.

Une introduction de gaz inerte par rintermédiaire d'un capillaire débouchant en fond de réacteur permet de réaliser un bullage pour obtenir une ébullition régulière.

Le réacteur comprend une double enveloppe pour réguler la température du milieu. Cette température est mesurée par une sonde disposée à l'intérieur du réacteur. Dans les essais ci-dessous, la température est maintenue constante à une valeur de 55°C, et l'installation est mise sous vide pour obtenir l'ébullition de l'eau.

Les essais ont été réalisés selon le mode opératoire suivant :

425g de cristaux de sel NYLON fabriqués industriellement par précipitation à partir d'une solution aqueuse sont ajoutés dans le réacteur avec 400 g d'eau.

Après agitation jusqu'à dissolution des cristaux, la température du milieu est régulée à 55°C et l'ébullition et évaporation de l'eau est entreprise par mise sous pression réduite.

La vitesse de rotation de l'agitation mécanique est fixée à 1000 tr/min.

La durée et vitesse de cristallisation sont déterminées par le débit de soutirage de l'eau évaporée. Ainsi, la cristallisation est arrêtée quand 200 ml d'eau ont été soutirés.

Le milieu réactionnel maintenu à une température supérieure à 35°C, est filtré. Le gâteau obtenu est lavé par de l'alcool absolu saturé en sel NYLON.

Le gâteau est ensuite séché pendant 12 heures par circulation d'air à température ambiante (environ 22°C) assurée par mise sous vide du gâteau.

Le sel NYLON obtenu est analysé par mesure de la distribution de taille des particules avec un granulomètre laser de dénomination commerciale MALVERN de type 2600 basé sur le principe de la diffraction de la lumière. La focale utilisée est de 1000 mm, les cristaux ayant été mis en suspension dans de l'éthanol saturé en sel NYLON.

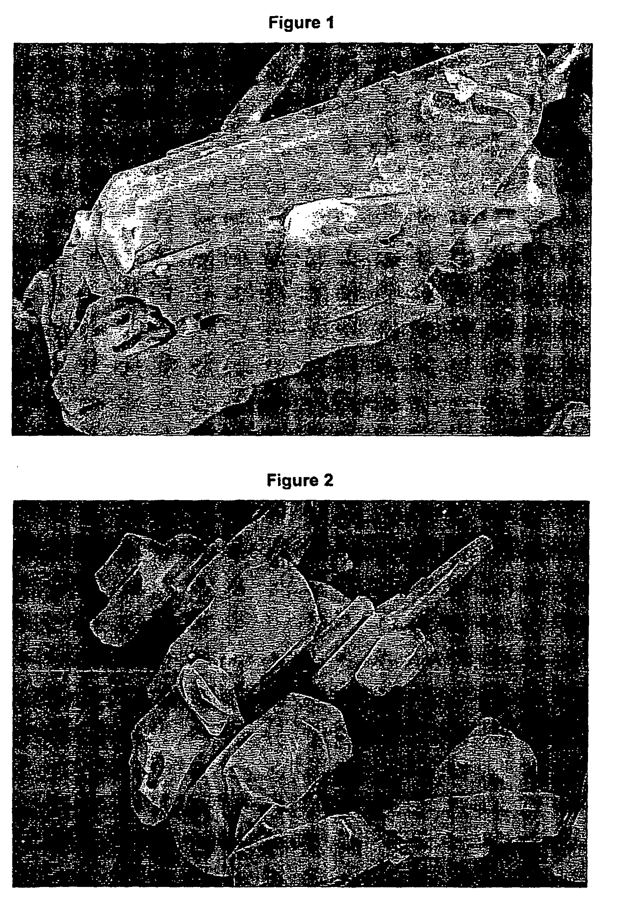

La forme des cristaux est également visualisée par microscopie à balayage électronique. Cette visualisation est illustrée par les figures 1 et 2 annexées.

Trois essais similaires ont été réalisés avec une puissance d'agitation ultrasonique égale respectivement à 0 W (sans ultrasons), 30 W et 100 W.

Les résultats obtenus sur la distribution de tailles des particules sont donnés dans le tableau ci-dessous:

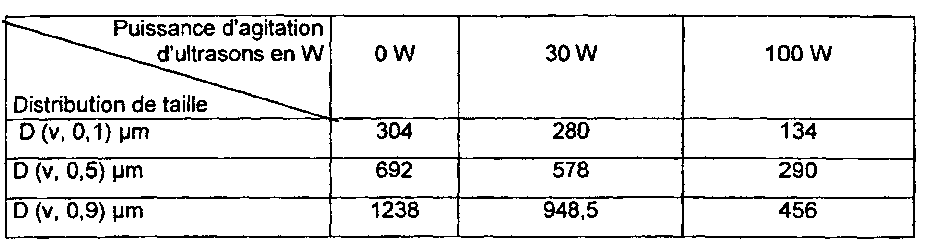

L'effet des ultrasons sur la taille des cristaux est constaté, mais également un resserrement de la distribution de taille pour l'agitation ultrasonique de puissance 100 W.

Les figures 1 et 2 annexées illustrent clairement l'effet de l'agitation ultrasonique sur la forme des cristaux de sel NYLON. En effet, la figure 1 montre que les cristaux obtenus sans agitation ultrasonique sont de plaquettes de forme irrégulière possédant des défauts de surface visibles tels que des trous et/ou des fissures. De plus, des agglomérats de cristaux sont visibles. Les cristaux illustrés par la figure 2 ont une forme de plaquette très régulière avec des faces marquées et très nettes et lisses. Par ailleurs, la forme des plaquettes est différente de celle observée sans agitation ultrasonique, la longueur de la plaquette est diminuée et son épaisseur augmentée. Les cristaux obtenus sous agitation ultrasonique apparaissent plus compacts et de forme plus régulière sans défaut de surface. Enfin, les agglomérats de cristaux ont presque totalement disparu.

En outre, des analyses de teneur en eau des cristaux obtenus ont été entreprises pour déterminer d'une part la teneur totale en eau et la quantité d'eau incluse dans les cristaux. Par eau incluse on entend l'eau qui n'est pas extractible par un solvant tel que le toluène et qui sous l'effet d'un séchage ne pourra être éliminée. Cette eau incluse est génératrice d'impuretés car elle a la composition des eaux mères de cristallisation, et très certainement elle favorise l'agglomération des granulés de sel NYLON au cours du stockage pour le transport, par exemple.

La teneur en eau totale est déterminée directement sur le sel NYLON après solubilisation du sel dans un solvant et analyse par la méthode KARL FISHER.

Pour déterminer la quantité d'eau incluse, on mesure la teneur en eau de surface en traitant 1 g de sel par 9 g de toluène déshydraté pour extraire l'eau de surface. La quantité d'eau extraite par le toluène est dosée par la méthode KARL FISHER.

Les résultats obtenus sont rassemblés dans le tableau ci-dessous:

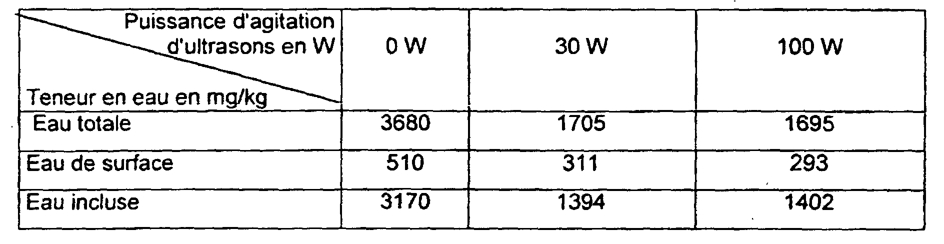

Ces résultats démontrent également l'effet signification de la présence de l'agitation ultrasonique au cours de la cristallisation.

## Revendications

1. Procédé de cristallisation de sel de diamine et de diacide carboxylique consistant à soumettre une solution aqueuse dudit sel à une agitation ultrasonique simultanément à une saturation de ladite solution aqueuse en sel.

2. Procédé selon la revendication 1, **caractérisée en ce que** la saturation de la solution aqueuse en sel est obtenue par refroidissement.

3. Procédé selon la revendication 1 ou 2, **caractérisé en ce que** la saturation de la solution aqueuse en sel est réalisée par évaporation de l'eau.

4. Procédé selon l'une des revendications précédentes, **caractérisé en ce que** la saturation de la solution aqueuse en sel est réalisée par addition simultanée ou successive de diacide carboxylique et de diamine dans ladite solution.

5. Procédé selon l'une des revendications précédentes, **caractérisé en ce que** la diamine est l'hexaméthylène diamine.

6. Procédé selon l'une des revendications précédentes, **caractérisé en ce que** le diacide carboxylique est l'acide adipique.

7. Procédé selon l'une des revendications précédentes, **caractérisé en ce que** la solution de sel comprend des cristaux naissants dudit sel.

8. Procédé selon l'une des revendications précédentes, **caractérisé en ce que** la fréquence de l'agitation ultrasonique est comprise entre 18 KHz et 200 KHz.

9. Procédé selon l'une des revendications précédentes, **caractérisé en ce que** la solution aqueuse est soumise à une agitation ultrasonique continue.

10. Procédé selon l'une des revendications 1 à 8, **caractérisé en ce que** la solution aqueuse est soumise à une agitation ultrasonique en mode pulsé.

11. Procédé selon l'une des revendications précédentes, **caractérisé en ce que** la solution aqueuse contenue dans un cristallisoir est mise en circulation dans un conduit en boucle fermée, l'agitation ultrasonique étant réalisée dans ledit conduit.

## Patentansprüche

1. Verfahren zur Kristallisation von Salz von Diamin und Dicarbonsäure, darin bestehend, daß man eine wäßrige Lösung des genannten Salzes gleichzeitig einem Rührvorgang mittels Ultraschall und einer Sättigung der genannten wäßrigen Lösung an Salz unterzieht.

2. Verfahren nach Anspruch 1, **dadurch gekennzeichnet, daß** die Sättigung der wäßrigen Lösung an Salz durch Abkühlung erhalten wird.

3. Verfahren nach Anspruch 1 oder 2, **dadurch gekennzeichnet, daß** die Sättigung der wäßrigen Lösung an Salz durch Verdampfen des Wassers realisiert wird.

4. Verfahren nach einem der vorstehenden Ansprüche, **dadurch gekennzeichnet, daß** die Sättigung der wäßrigen Lösung an Salz durch gleichzeitige oder aufeinanderfolgende Zugabe von Dicarbonsäure und Diamin zu der genannten Lösung realisiert wird.

5. Verfahren nach einem der vorstehenden Ansprüche, **dadurch gekennzeichnet, daß** das Diamin Hexamethylendiamin ist.

6. Verfahren nach einem der vorstehenden Ansprüche, **dadurch gekennzeichnet, daß** die Dicarbonsäure Adipinsäure ist.

7. Verfahren nach einem der vorstehenden Ansprüche, **dadurch gekennzeichnet, daß** die Lösung des Salzes Keim-Kristalle des genannten Salzes umfaßt.

8. Verfahren nach einem der vorstehenden Ansprüche, **dadurch gekennzeichnet, daß** die Frequenz des Ultraschallrührens zwischen 18 kHz und 200 kHz liegt.

9. Verfahren nach einem der vorstehenden Ansprüche, **dadurch gekennzeichnet, daß** die wäßrige Lösung einem kontinuierlichen Ultraschallrühren unterzogen wird.

10. verfahren nach einem der Ansprüche 1 bis 8, **dadurch gekennzeichnet, daß** die wäßrige Lösung einem Ultraschallrühren in gepulster Form unterzogen wird.

11. Verfahren nach einem der vorstehenden Ansprüche, **dadurch gekennzeichnet, daß** die wäßrige Lösung, die in einem Kristallisator enthalten ist, im Kreislauf in einer Leitung in geschlossener Schleife geführt wird, wobei das Ultraschallrühren in dieser genannten Leitung realisiert wird.

## Claims

1. Process for crystallizing a salt of a diamine and of a dicarboxylic acid, which consists in subjecting an aqueous solution of the said salt to ultrasonic agitation while simultaneously saturating the said aqueous solution with salt.

2. Process according to Claim 1, **characterized in that** the aqueous solution is saturated with salt by cooling.

3. Process according to Claim 1 or 2, **characterized in that** the aqueous solution is saturated with salt by evaporating water.

4. Process according to one of the preceding claims, **characterized in that** the aqueous solution is saturated with salt by simultaneous or successive addition of dicarboxylic acid and of diamine to the said solution.

5. Process according to one of the preceding claims, **characterized in that** the diamine is hexamethylenediamine.

6. Process according to one of the preceding claims, **characterized in that** the dicarboxylic acid is adipic acid.

7. Process according to one of the preceding claims, **characterized in that** the salt solution comprises seed crystals of the said salt.

8. Process according to one of the preceding claims, **characterized in that** the ultrasonic agitation frequency is between 18 KHz and 200 KHz.

9. Process according to one of the preceding claims, **characterized in that** the aqueous solution is subjected to a continuous ultrasonic agitation.

10. Process according to one of Claims 1 to 8, **characterized in that** the aqueous solution is subjected to an ultrasonic agitation in pulsed mode.

11. Process according to one of the preceding claims, **characterized in that** the aqueous solution contained in a crystallizer is circulated in a closed-loop pipe, the ultrasonic agitation being carried out in the said pipe
